(19) Europäisches Patentamt
European Patent Office
Office européen des brevets

(11) **EP 4 249 912 A1**

(12) **EUROPEAN PATENT APPLICATION**

(43) Date of publication:
**27.09.2023 Bulletin 2023/39**

(21) Application number: **22164038.6**

(22) Date of filing: **24.03.2022**

(51) International Patent Classification (IPC):
**G01N 33/50** (2006.01)          **G01N 33/68** (2006.01)

(52) Cooperative Patent Classification (CPC):
**G01N 33/5008; G01N 33/68;** G01N 2333/4748;
G01N 2500/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Cleara Biotech B.V.
3584 CM Utrecht (NL)**
• **UMC Utrecht Holding B.V.
3584 CS Utrecht (NL)**

(72) Inventors:
• **DE KEIZER, Peterus, Leonardus, Josephus
3524WJ Utrecht (NL)**

• **MADL, Tobias
8010 Graz (AT)**
• **LEHMANN, Johannes
3012JM Rotterdam (NL)**
• **PUTAVET, Diana
3031EA Rotterdam (NL)**
• **BAAR, Marjolein, Petronella
1222LN Hilversum (NL)**

(74) Representative: **Krauss, Jan
SKM-IP PartGmbB
Oberanger 45
80331 München (DE)**

Remarks:
The complete document including Reference
Table(s) and the Sequence Listing(s) can be
downloaded from the EPO website

(54) **PHOSPHORYLATION OF P53 AS A PROGNOSTIC OR DIAGNOSTIC MARKER FOR THE TREATMENT OF SENESCENT CELLS IN A MAMMAL**

(57) The present invention relates to a method, in particular an *in vitro* method, for identifying an improved anti-senescence compound based on detecting the binding of said compound in the presence of at least one phosphorylated amino acid in the transcription activation domain (TAD) of mammalian protein p53. The present invention further relates to a method, in particular an *in vitro* method, for monitoring an anti-senescence treatment or prophylaxis in a mammalian subject in need thereof, based on detecting the amount of phosphorylation of amino acids in the TAD of the mammalian p53 protein in a biological sample obtained from said subject and/or detecting the amount of the promyelocytic leukemia protein (PML) bodies in a biological sample obtained from said subject. Furthermore, the present invention relates to a kit for performing the above methods as well as respective uses thereof. Finally, improved anti-senescence compounds or pharmaceutical compositions are provided.

EP 4 249 912 A1

**Description**

The present invention relates to a method, in particular an *in vitro* method, for identifying an improved anti-senescence compound based on detecting the binding of said compound in the presence of at least one phosphorylated amino acid in the transcription activation domain (TAD) domain of mammalian protein p53. The present invention further relates to a method, in particular an *in vitro* method, for monitoring an anti-senescence treatment or prophylaxis in a mammalian subject in need thereof, based on detecting the amount of phosphorylation of amino acids in the TAD of the mammalian p53 protein in a biological sample obtained from said subject and/or detecting the amount of the promyelocytic leukemia protein (PML) bodies in a biological sample obtained from said subject. Furthermore, the present invention relates to a kit for performing the above methods as well as respective uses thereof. Finally, improved anti-senescence compounds or pharmaceutical compositions are provided.

**Background of the invention**

Apoptosis (programmed cell death) provides for the killing of damaged cells. Dying cells that undergo the final stages of apoptosis display phagocytic molecules, marking these cells for phagocytosis by cells possessing the appropriate receptors, such as macrophages.

It has been shown that senescent cells accumulate with age, and at sites of age-related pathology. Further, senescent cells can acquire mutations that allow them to re-enter a proliferative state. Benign senescent lesions thus retain the capacity to become malignant. Indeed, senescence has been associated with a plethora of (age-related) pathologies and, conversely, genetic clearance of senescent cells can delay features of aging.

Cellular senescence is a stress response that in many cases elicits a permanent cell cycle arrest and triggers profound phenotypic changes such as the production of a bioactive secretome, referred to as the senescence-associated secretory phenotype (SASP). Acute senescence induction protects against cancer and limits fibrosis, but lingering senescent cells drive age-related disorders. Thus, targeting senescent cells to delay aging and limit dysfunction, known as "senotherapy," is gaining momentum. While drugs that selectively kill senescent cells, termed "senolytics" are a major focus, SASP-centered approaches are emerging as alternatives to target senescence-associated diseases.

It was recently shown that clearance of senescent cells in a genetic fashion could markedly improve the fitness and decrease parameters of aging in a mouse model for accelerated aging. These mice showed reduced signs of aging measured by kyphosis (excessive bone curvature), muscle strength, fat deposition and cataracts. This provided further evidence that cellular senescence and the SASP is causally linked to age-associated phenotypes and cancer (Baker et al., 2011. Nature 479(7372):232-6). This proof-of-concept evidence was obtained in a genetic fashion that has poor therapeutic applicability.

Studies have consistently revealed FOXO (Forkhead box O) transcription factors as important determinants in aging and longevity. FOXO proteins represent a subfamily of transcription factors conserved from *Caenorhabditis elegans* to mammals that act as key regulators of longevity downstream of insulin and insulin-like growth factor signaling. Invertebrate genomes have one FOXO gene, while mammals have four FOXO genes: FOXO1, FOXO3, FOXO4, and FOXO6. In mammals, this subfamily is involved in a wide range of crucial cellular processes regulating stress resistance, metabolism, cell cycle arrest, and apoptosis. Their role in longevity determination is complex and remains to be fully elucidated.

Bourgeois and Madl (in: Regulation of cellular senescence via the FOXO4-p53 axis. FEBS Lett. 2018 Jun;592(12):2083-2097) disclose that in the last decade both FOXO and p53 have been identified as key players in aging, and their misregulation is linked to numerous diseases including cancers. However, many of the underlying molecular mechanisms are said to remain mysterious, including regulation of aging by FOXOs and p53. Several activities appear to be shared between FOXOs and p53, including their central role in the regulation of cellular senescence. They focus on the recent advances on the link between FOXOs and p53, with a particular focus on the FOXO4-p53 axis and the role of FOXO4/p53 in cellular senescence. Potential strategies for targeting the FOXO4-p53 interaction to modulate cellular senescence as a drug target in treatment of aging-related diseases and morbidity are discussed.

Miller Jenkins et al. (in: p53 N-terminal phosphorylation: a defining layer of complex regulation, Carcinogenesis, Volume 33, Issue 8, August 2012, Pages 1441-1449, https://doi.org/10.1093/carcin/bgs145) disclose that the p53 tumor suppressor is a critical component of the cellular response to stress. As it can inhibit cell growth, p53 is mutated or functionally inactivated in most tumors. A multitude of protein-protein interactions with transcriptional cofactors is central to p53-dependent responses. In its activated state, p53 is extensively modified in both the N- and C-terminal regions of the protein. These modifications, especially phosphorylation of serine and threonine residues in the N-terminal transcription activation domain, affect p53 stability and activity by modulating the affinity of protein-protein interactions. They review recent findings from *in vitro* and *in vivo* studies on the role of p53 N-terminal phosphorylation. These modifications can either positively or negatively affect p53 and add a second layer of complex regulation to the divergent interactions of the p53 transcription activation domain.

Feng et al. (in: Ser46 phosphorylation regulates p53-dependent apoptosis and replicative senescence. Cell Cycle. 2006 Dec;5(23):2812-9. doi: 10.4161/cc.5.23.3526. Epub 2006 Dec 1. PMID: 17172844) disclose that cell line studies have shown that phosphorylation of Ser46 is correlated with the activation of p53 apoptotic activity.

WO 2016/118014 relates to a peptide comprising the amino acid sequence LTLRKEPASEIAQSILEAYSQNGWANRRSGGKRP (SEQ ID NO: 7), wherein the amino acids in said amino acid sequence are D-amino acid residues, and to methods for the use of this peptide in the treatment of age-related disorders. The peptide exhibits apoptosis-inducing activity in senescent cells or cells having an increased FOXO4 expression as compared to a control cell and expressing Ser15 phosphorylated p53 (pSer15-p53).

WO 2013/152038 relates to uses of agents that inhibit Jun kinases and/or FOXO4 in treating cancer and/or removing senescent cells in an individual. In some embodiments, the agent is a small molecule such as SP600125. In some embodiments, the agent is a small molecule such as AS601245. In some embodiments, the agent is a peptide as disclosed.

WO 2013/152041 relates to agents that inhibit FOXO4 function and uses thereof in treating cancer and/or removing senescent cells in an individual. In some embodiments, the agent that inhibits FOXO4 is a peptide that inhibits FOXO4 function in a cell, wherein the peptide comprises an amino acid sequence that has at least 80% identity to a fragment of the FOXO4 as disclosed.

WO 2018/129007 relates to conditionally active proteins that target senescent cells and to methods of generating such conditionally active proteins.

WO 2021/165538 relates to improved compounds for use in the treatment of diseases or conditions where the removal of senescent cells, scarred cells, and/or cancerous cells is beneficial, for example cancer. WO 2021/165538 also relates to methods of treating an individual suffering, or suspected of suffering, from a disease or condition wherein the removal of senescent cells, scarred cells, and/or cancerous cells is beneficial.

It is clear from the above that there is still a need in the art for improved assays and methods that identify effective treatments of conditions using compounds that selectively induce apoptosis in senescent cells. The cellular senescence is particularly linked to degenerative (loss-of-function) diseases and cancer (gain of function), which both involve the mammalian protein p53. It is therefore an object of the present invention, to provide assays and methods that identify effective treatments of conditions using compounds that selectively induce apoptosis in senescent cells.

In a first aspect thereof, the present invention solves the above problem by providing a method for identifying an improved anti-senescence compound, comprising the steps of: a) contacting at least one anti-senescence candidate molecule with a transcription activation domain (TAD) of a mammalian p53 protein, b) detecting a specific binding of the candidate molecule to the TAD in the presence of at least one phosphorylated amino acid in the TAD, and c) comparing said specific binding to the binding in the absence of the at least one phosphorylated amino acid in the TAD, wherein an increase of the binding in the presence of the at least one phosphorylated amino acid in the TAD identifies an improved anti-senescence compound.

Preferred is the method according to the present invention, wherein the phosphorylation of said TAD1 is positioned at Ser15 and/or Ser20 of human p53, and/or the phosphorylation of said TAD2 is positioned at Ser46 and/or Thr55 of human p53, or the analog positions in other mammalian p53 TADs. Preferably, the phosphorylation of said TAD2 is positioned at Thr55 of human p53, or the analog positions in other mammalian p53 TADs. In another preferred embodiment of the method according to the present invention, detecting the binding furthermore comprises, when possible, detecting phosphorylation of Ser392 of human p53 or the analog positions in other mammalian p53 proteins, and comparing said specific binding to the absence of said phosphorylated amino acid.

In a second aspect thereof, the present invention solves the above problem by providing an anti-senescence compound as identified according to a method according to the present invention, or a pharmaceutical composition comprising said anti-senescence compound, together with a pharmaceutically acceptable carrier.

In a third aspect thereof, the present invention solves the above problem by providing a method for monitoring an anti-senescence treatment or prophylaxis in a mammalian subject in need thereof, comprising a) providing an anti-senescence treatment or prophylaxis to said subject, comprising administering to said subject an anti-senescence compound that specifically binds to the TAD of the p53 protein or a pharmaceutical composition comprising said anti-senescence compound, preferably a compound or pharmaceutical composition according to the present invention, b) detecting the amount of phosphorylation of amino acids in the TAD of the p53 protein in a biological sample obtained from said subject and/or detecting the amount of the promyelocytic leukemia protein (PML) bodies in a biological sample obtained from said subject, and c) comparing the amount(s) as detected in step b) with the amount in an earlier sample taken from said subject, and/or a control sample.

In a fourth aspect thereof, the present invention solves the above problem by providing a method for predicting or prognosing the success of, progress of and/or sensitivity for an anti-senescence treatment or prophylaxis in a mammalian subject, comprising performing the method according to the present invention, wherein an increase of the phosphorylation and/or PML protein is indicative for the success of, progress of and/or sensitivity for the anti-senescence treatment or prophylaxis in the mammalian subject.

In a fifth aspect thereof, the present invention solves the above problem by providing an anti-senescence compound that specifically binds to the TAD of the p53 protein or a pharmaceutical composition comprising said anti-senescence compound, preferably a compound or pharmaceutical composition according to the present invention for use in the prophylaxis or treatment of senescent cells, wherein the senescent cells exhibit an increase of the amount of phosphorylation of Ser46 and/or Thr55 of human p53, or the analog positions in other mammalian p53 TADs, and/or an increase of the amount of PML protein, when compared to a control cell.

Preferred is an anti-senescence compound or a pharmaceutical composition for use according to the present invention, wherein the senescent cells further exhibit an increase of the amount of phosphorylation of Ser392 of human p53 or the analog positions in other mammalian p53 proteins, when compared to a control cell. Further preferred is the anti-senescence compound or a pharmaceutical composition for use according to the present invention, wherein said senescent cells are selected from primary tumor cells, metastatic tumor cells, cells of secondary tumors, cells of micro-metastases, fibrotic cells, breast cancer cells, such as triple-negative breast cancer cells, lung cancer, ovarian cancer, pancreatic cancer and/or liver cancer cells or metastatic cells thereof.

In a sixth aspect thereof, the present invention solves the above problem by providing a method for preventing or treating senescent cells in a subject, wherein the senescent cells exhibit an increase of the amount of phosphorylation of Ser46 and/or Thr55 of human p53, or the analog positions in other mammalian p53 TADs, and/or an increase of the amount of PML protein, bodies when compared to a control cell, comprising administering to said subject an effective amount of an anti-senescence compound that specifically binds to the TAD of the p53 protein or a pharmaceutical composition comprising said anti-senescence compound, preferably a compound or pharmaceutical composition according to the present invention.

Preferred is the method according to the present invention, wherein the senescent cells further exhibit an increase of the amount of phosphorylation of Ser392 of human p53 or the analog positions in other mammalian p53 proteins, when compared to a control cell. Further preferred is a method, wherein said senescent cells are selected from primary tumor cells, metastatic tumor cells, cells of secondary tumors, cells of micro-metastases, fibrotic cells, breast cancer cells, such as triple-negative breast cancer cells, lung cancer, ovarian cancer, pancreatic cancer and/or liver cancer cells or metastatic cells thereof.

The present invention is based on the surprising finding by the inventors, that the phosphorylation of the p53 protein is an important factor for the effective binding of anti-senescence molecules to the TADs of p53. The TAD1 and 2 contain four amino acid residues that can change in charge due to phosphorylation. FOXO4-FH derived binders as disclosed herein specifically interact with TAD2, and this indicates S46 and T55 are important for binding and selectivity.

In the context of the above, the present invention analyzed the efficiency of binding of certain anti-senescence molecules, such as retro-inverso peptides, derived from the mammalian, and in particular human, Forkhead box protein O4 (FOXO4), wherein the amino acids in said amino acid sequence are D-amino acid residues, to the p53 protein. Preferred examples are compounds comprising the following amino acid sequences: RKKASSKIEAAILDAFSQNWRFFKRPPRRRQRRKKRG (SEQ ID NO: 1; designated as CL04124); RKKASSKIEAEILDAFSQNWRRKRPPRRRQRRKKRG (SEQ ID NO: 2; designated as CL04177); and AKIEAAILDAFSQNWRKRRRRQRRKKRG (SEQ ID NO: 3; designated as CL04183).

It was furthermore and also independently from the above found that binding of the anti-senescence molecules is further strongly influenced by the phosphorylation of the p53 protein at position S392, and it was found that S392 phosphorylation causes p53 to adopt an "open" conformation, and certain preferred anti-senescence molecules as tested, such as the molecule CL04183 are especially effective against cells with pS392/"open"- p53.

Promyelocytic leukemia protein (PML) (also known as MYL, RNF71, PP8675 or TRIM19) is the protein product of the PML gene. PML protein is a tumor suppressor protein required for the assembly of a number of nuclear structures, called PML-nuclear bodies or PML (protein) bodies, which form amongst the chromatin of the cell nucleus. These nuclear bodies are present in mammalian nuclei, at about 1 to 30 per cell nucleus. PML-(N)Bs are known to have a number of regulatory cellular functions, including involvement in programmed cell death, genome stability, antiviral effects and controlling cell division. PML mutation or loss, and the subsequent dysregulation of these processes, has been implicated in a variety of cancers.

In addition to the above, in the context of the present invention it was surprisingly found that PML bodies are useful as a "proxy-p53-biomarker" for the efficacy of certain preferred anti-senescence molecules as tested, such as the molecule CL04183. CL04183 was found to be especially effective against cells with pS392/"open"- p53 and PML elevation, in particular against PML$^{high}$ metastases in liver and lung. This is probably due to the fact that metastases require initiating stem-like cells, and PML is associated with a state of "sternness", esp. CD44 and active β-Catenin.

Diana Putavet et al. (in: Repurposing the FOXO4 senolytic against triple-negative breast cancer [abstract]. In: Proceedings of the 2021 San Antonio Breast Cancer Symposium; 2021 Dec 7-10; San Antonio, TX. Philadelphia (PA): AACR; Cancer Res 2022;82(4 Suppl):Abstract nr P1-19-02) disclose that Triple-Negative Breast Cancer (TNBC) has tan especially poor prognosis partly due to these tumors lacking relevant molecular targets. The most commonly mutated gene in TNBC is the tumor suppressor p53. They found that, in TNBCs, mutant p53 attained a distinctive conformation that has novel oncogenic roles through binding to the transcription factor Forkhead box O (FOXO) 4 sequestered within promyelocytic leukemia (PML) foci. Since these nuclear structures are specific to senescent cells, we tested the senolytic FOXO4 peptide. In cytotoxicity experiments, we found this compound to target TNBCs specifically over other breast cancer subtypes. Most importantly, these compounds decrease metastatic burden in the most commonly-used mouse model for human breast cancer metastasis. They demonstrate that mutant p53-driven cancers presented senescent cell-specific characteristics that makes them a great candidate for the FOXO4-directed anti-senescence therapy. Creative repurposing of senolytics may translate to other types of cancer that are driven by mutant p53. Phosphorylation of p53 is not mentioned.

In the context of the present invention, the term "TP53 or "p53" shall designate the mammalian protein p53, in particular the genes for human TP53, mouse Trp53, rat Tp53, monkey, sheep, goat, hamster, dog, or cat TP53. Included are also all natural variants, and functional fragments of p53 that bind to FOXO4 protein of fragments thereof, in particular the trans activation domain (TAD) or transcriptional activation domains. The amino acid sequence of human p53 can be found at P04637.

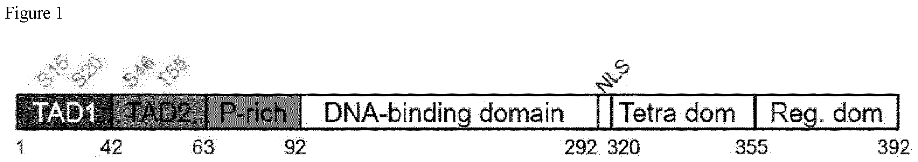

In the context of the present invention, the term "TAD" shall relate to one of the two distinct trans activation domains as harbored at the N-terminus of p53 protein as above. Raj and Attardi (in: The Transactivation Domains of the p53 Protein. Cold Spring Harb Perspect Med. 2017;7(1):a026047. Published 2017 Jan 3. doi:10.1101/cshperspect.a026047) disclose the structure and function of TADs of p53 (see in particular Figure 1). Disclosed are also serines and threonines in the p53 TADs (about positions 1-85/92) that are targets of phosphorylation and affect p53 TAD interactions with binding partners. Human TAD1 is at about amino acids 1-40/42, and TAD2 is at about amino acids 41/43-63 or 92, when including the carboxy-terminal portion of the region now considered the proline-rich domain (PRD), see also Figure 1.

Therefore, as mentioned above, in a first aspect thereof, the present invention solves the above problem by providing a method, in particular an in vitro method, for identifying an improved anti-senescence compound, comprising the steps of: a) contacting at least one anti-senescence candidate molecule with a transcriptional activation domain (TAD) of a mammalian p53 protein, b) detecting a specific binding of the candidate molecule to the TAD in the presence of at least one phosphorylated amino acid in the TAD, and c) comparing said specific binding to the binding in the absence of the at least one phosphorylated amino acid in the TAD, wherein an increase of the binding in the presence of the at least one phosphorylated amino acid in the TAD identifies an improved anti-senescence compound.

Preferred is the method according to the present invention, wherein said contacting is in vivo or in vitro, in solution or comprises the TAD of a mammalian p53 protein or the anti-senescence candidate molecule bound or conjugated to a solid carrier. Respective formats are also described in the art, and known to the person of skill.

Preferred is the method according to the present invention, wherein the phosphorylation of said TAD1 is positioned at Ser15 and/or Ser20 of human p53, and/or the phosphorylation of said TAD2 is positioned at Ser46 and/or Thr55 of human p53, or the analog positions in other mammalian p53 TADs. Preferably, the phosphorylation of said TAD2 is positioned at Thr55 of human p53, or the analog positions in other mammalian p53 TADs. In another preferred embodiment of the method according to the present invention, detecting the binding furthermore comprises, when possible, detecting phosphorylation of Ser392 of human p53 or the analog positions in other mammalian p53 proteins, and comparing said specific binding to the absence of said phosphorylated amino acid.

In the context of the present invention, any suitable mammalian TAD may be used. Preferred is the method according to the present invention, wherein the TAD of the mammalian p53 protein is selected from TAD1 and/or TAD2 of human, mouse, rat, monkey, sheep, goat, hamster, dog, and cat p53 protein, optionally as part of the full-length p53 protein, in particular a recombinant p53 protein, a full-length p53 protein comprising phosphorylation at Ser392 of human p53 or the analog positions in other mammalian p53 proteins, a recombinant fusion protein comprising said TAD1 and/or TAD2, or a phosphorylated fragment of the TAD1 or TAD2, and mutated variants thereof. Preferably, the phosphorylation of said TAD1 domain is positioned at Ser15 and/or Ser20 of human p53, and/or the phosphorylation of said TAD2 is positioned at Ser46 and/or Thr55 of human p53, or the analog positions in other mammalian p53 TADs.

In the context of the present invention, the anti-senescence candidate molecule can be selected from any suitable molecule that is suitable to bind the mammalian p53, such as a chemical organic molecule, a molecule selected from a library of small organic molecules (molecular weight less than 500 Da), a molecule selected from a combinatory library, a cell extract, in particular a plant cell extract, a small molecular drug, a protein, a protein fragment, a molecule selected from a peptide library, an antibody or fragment thereof. Particularly preferred are a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124 and CL04177 as explained above. Other peptides are disclosed in WO 2021/165538 (incorporated by reference). These candidate molecules are used as a basis to screen for improved compounds, wherein certain amino acids are modified (see below).

If the anti-senescence candidate molecule is a peptide, the compound may further comprise a sequence conferring cell-penetrating properties, organelle targeting properties, nuclear localization, mitochondrial localization, blood brain barrier permeability, cell membrane localization, and/or peptidase cleavage, such as, for example, the TAT-sequence of HIV (GRKKRRQRRRPP, SEQ ID NO: 8), or ARKKRRQRRRPPP (SEQ ID NO: 9). If the anti-senescence candidate molecule is a peptide, the compound may also further comprise non-naturally occurring amino acids, preferably having the same or substantially the same desired properties of the natural amino acids in that position of said compound, modified amino acids, such as, for example, linker amino acids, staples, cysteine bridges, glycolysation sites, ubiquitination and/or pegylation sites.

In the context of the present invention, any method that is suitable for detecting the binding of the compound may be used. Respective methods are known to the person of skill and are disclosed in the art. Preferred is the method according to the present invention, wherein detecting said binding comprises detecting phosphorylation comprising radiolabeled 32 P-orthophosphate, phospho-specific antibodies, and/or mass spectrometry.

The components of the assays as disclosed herein may be labelled, for example with a radiolabel or fluorescent label or with an antigenic label.

In another aspect of the invention, detecting the binding comprises a competitive binding of the compound, in particular a peptide, such as CL04177, CL04124 or CL04183, with the protein FOXO4 or a TAD binding fragment thereof, i.e., tests for the properties of said modified compound in the presence of said at least one compound, compared to the absence of said compound, and the differnt states of phophorylation thereof.

Detecting the binding may furthermore comprises, when possible, detecting phosphorylation of Ser392 of human p53 or the analog positions in other mammalian p53 proteins, and comparing said specific binding to the absence of said phosphorylated amino acid.

Yet another aspect of the present invention then relates to a method for identifying an improved compound that binds to the TAD of p53 or a FOXO4 binding fragment thereof or binds to p53 and inhibits the interaction of FOXO4 with p53 in a cell, comprising the steps of a) providing at least one anti-senescence candidate compound; b) suitably modifying said compound of a); c) contacting at least one anti-senescence candidate molecule of b) with a transcriptional activation domain (TAD) of a mammalian p53 protein; d) detecting a specific binding of the candidate molecule to the TAD in the presence of at least one phosphorylated amino acid in the TAD; and e) comparing said specific binding to the binding in the absence of the at least one phosphorylated amino acid in the TAD, wherein an increase of the binding in the presence of the at least one phosphorylated amino acid in the TAD identifies an improved anti-senescence compound.

Preferred is the method according to the present invention, further comprising testing said compound as identified for its activity to induce apoptosis and/or to kill senescent, scarred and/or tumor cells, preferably comprising determination of elevated caspase-3/7 activity, loss of mitochondrial cytochrome C, TUNEL positivity, extracellular annexin- V positivity, and/or and elevation in cell death markers, such as cellular propidium iodide inclusion, lactate-dehydrogenase release assay, and/or loss of viability, such as loss of incorporation of calcein-AM or an MTS viability assay.

Further preferred is the method according to the present invention, wherein said TAD or said FOXO4 binding fragment thereof is recombinantly expressed in a cell.

The present invention further relates to improved inhibiting compounds that are designed since the evolutionary differences between FOXO 1/3/6 and FOXO4 indicated that several amino acids can be changed or modified in order to create improved inhibiting compounds. All these modifications present a new strategy wherein any one of these or a combination thereof finally lead to the "rational design" of improved molecules for use in the context of the present invention. The present invention also includes strategies in order to further improve compounds that have only partially undergone "directed evolution" or "directed mutagenesis".

In the course of the "rational design" of improved molecules for use in the context of the present invention, and in order to further improve compounds that have only partially undergone "directed evolution" or "directed mutagenesis", the compounds can undergo several successive rounds of the above methods.

Following the provision of a compound (e.g., a peptide), for example after suitable chemical synthesis thereof, the compound can be modified. In general, many methods of how to modify compounds of the present invention are known to the person of skill, and are disclosed in the literature. Modifications of the compounds will usually fall into several categories, for example a) mutations/changes of amino acids into different amino acids, b) chemical modifications of amino acids, e.g., through the addition of additional chemical groups, c) modifications of the amino acid structure (e.g., L- into D-form) or bonds (e.g., introduction of retro-inverso bonds), d) changes of the length of the compound, and e) the attachment of additional groups to the molecule (including marker groups, labels, linkers or carriers, such as chelators).

As above, the evolutionary differences between FOXO1/3/6 and FOXO4 indicate that several amino acids can still be modified in order to create improved compounds showing the desired binding and - ultimately - also inhibiting activity. An analysis of the FOX04-FH fragment and the p53-TAD2 domain shows that negative charges in p53 mediate binding to FOXO4, as well as a hydrophobic Tyr. Therefore, amino acids of the peptides according to the present invention can be mutated into positive amino acids, as beneficial for the strength of the interaction. Examples are lysine (K), arginine (R), and histidine (H). The NMR experiments also showed that negatively charged amino acids in p53 are responsible for the interaction with FOXO4 around the SQ motif (site). This site can become phosphorylated, and to prevent this, the site can be mutated to a small amino acid, like alanine (A), glycine (G) or serine (S). Methods for introducing such mutations are known to the person of skill, and include the introduction of changes during chemical synthesis of the peptide, or genetic methods whereby the peptide encoding nucleotide sequence is altered accordingly, e.g., by oligonucleotide-based mutagenesis, mutagenesis comprising PCR, or the like. Also a random mutagenesis is possible.

Furthermore, amino acids can be altered by chemical modifications, e.g., through the addition of additional chemical groups during synthesis or via post-translational or post synthesis modifications. Methods to modify amino acids are well known in the state of the art, and summarized, for example, in Christopher D. Spicer & Benjamin G. Davis. Selective chemical protein modification Nature Communications volume 5, Article no.: 4740 (2014); or Sakamoto S, Hamachi I. Recent Progress in Chemical Modification of Proteins. Anal Sci. 2019 Jan 10;35(1):5-27. Also, modifications of the amino acid structure (e.g., L- into D-form) or the bonds between the amino acids (e.g., introduction of retro-inverso bonds during synthesis) can be performed.

Finally, additional chemical and/or functional groups to the molecule, such as, for example, marker groups, labels, linker amino acids, staples, cysteine bridges, glycolysation sites, ubiquitination and/or pegylation sites, linkers or carriers, such as chelators.

In a next step, the modified compound is tested for at least one of binding of said at least one compound to the TAD of p53 under different states of phophorylation thereof. As discussed herein, the property of the compound to bind to TAD and the state of phophorylation thereof is essential for all uses of the compound, be they therapeutic or diagnostic. In the context of the present invention, an "improved" binding comprises both scenarios where the modified compound binds to the same extent as the unmodified (i.e., starting) compound, although the compound has been modified (e.g., by dimerization or by adding markers or other groups). Preferred is a compound as modified that exhibits a stronger binding to the target, i.e., the TAD of p53, in the state of phophorylation thereof. Also preferred is a compound that shows a longer binding to the target, i.e., the TAD or a binding fragment thereof, for example because of an improved stability of said modified compound in vitro or in vivo.

Assays to detect binding of the compound to the target (i.e., the TAD) are well known to the person of skill and preferably include mass spectrometry, NMR assays, pull-down assays, or the like.

As mentioned above, the property of the compound to bind to the TAD and the state of phophorylation thereof is essential for all uses of the compound, be they therapeutic or diagnostic. Ideally, the binding is also specific or at least substantially or essentially specific for the intended target, i.e., the TAD. This will reduce or avoid any unwanted side effects in the medical use of the compound and reduce or avoid any background or false positive results in a diagnostic use.

In the last step of the method, an improved compound is identified that - in the preferred aspect of the present invention - binds to the TAD of p53, in particular to the TAD2 of p53, depending on the state of phophorylation thereof and preferably binds to the TAD depending on the state of phophorylation thereof and inhibits the interaction of FOXO4 with p53 in a cell based on said determining when compared to a compound as provided at the beginning of the method. While this function is deemed essential for the therapeutic function of the compounds according to the present invention by eliminating senescent cells, improved diagnostic molecules according to the present invention may not mandatorily require such property/function.

The identification of such improved compounds may include to detect the potency of these compounds, such as FOXO4-peptides, to better bind the phosphorylated TAD and eliminate senescent cells, and optionally the identification of structural requirements that determine this property; and/or to detect the selectivity of these compounds, such as FOXO4-derved peptides, to eliminate senescent cells, and again optionally the identification of structural requirements that determine this property.

Preferably, an improved compound, such as a peptide or improved peptide according to the invention is considered to exhibit apoptosis-inducing activity in senescent cells if it kills, clears, removes or reduces the viability of at least 5, 10, 15, 20, 25, 30, 40, 50, 60, 70 or 80% of the cells in a senescent cell culture. Preferably, a peptide according to the invention selectively exhibits apoptosis-inducing activity in senescent cells, i.e., not in non-senescent cells. A peptide according to the invention favors apoptosis in senescent cells over apoptosis in non-senescent cells by at least a factor 1.1, 1.2, 1.3, 1.4, 1.5, 1.6, 1.7, 1.8, 1.9, 2.0, 2.5, 3, 4, 5 or higher.

A peptide compound according to the invention is preferably isolated. A peptide compound according to the invention is preferably produced using suitable peptide synthesis.

Yet another aspect of the present invention then relates to an improved anti-senescence compound as identified according to a method according to the present invention, or a pharmaceutical composition comprising said anti-senescence compound, together with a pharmaceutically acceptable carrier.

Pharmaceutical compositions as used may optionally comprise a pharmaceutically acceptable carrier. Pharmaceutically acceptable carriers or excipients include diluents (fillers, bulking agents, e.g., lactose, microcrystalline cellulose), disintegrants (e.g., sodium starch glycolate, croscarmellose sodium), binders (e.g., PVP, HPMC), lubricants (e.g., magnesium stearate), glidants (e.g., colloidal $SiO_2$), solvents/co-solvents (e.g., aqueous vehicle, Propylene glycol, glycerol), buffering agents (e.g., citrate, gluconates, lactates), preservatives (e.g., Na benzoate, parabens (Me, Pr and Bu), BKC), antioxidants (e.g., BHT, BHA, Ascorbic acid), wetting agents (e.g., polysorbates, sorbitan esters), thickening agents (e.g., methylcellulose or hydroxyethylcellulose), sweetening agents (e.g., sorbitol, saccharin, aspartame, acesulfame), flavoring agents (e.g., peppermint, lemon oils, butterscotch, etc.), humectants (e.g., propylene, glycol, glycerol, sorbitol). Other suitable pharmaceutically acceptable excipients are *inter alia* described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for peptides and will readily be able to choose suitable pharmaceutically acceptable carriers or excipients, depending, e.g., on the formulation and administration route of the pharmaceutical composition.

The pharmaceutical composition can be administered orally, e.g., in the form of pills, tablets, coated tablets, sugar coated tablets, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g., in the form of ointments, creams or tinctures.

In addition to the aforementioned compounds of the invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds of the invention and also other therapeutically active substances as described herein.

Yet another aspect of the present invention then relates to a method, such as diagnostic method, for monitoring an anti-senescence treatment or prophylaxis in a mammalian subject in need thereof, comprising a) providing an anti-senescence treatment or prophylaxis to said subject, comprising administering to said subject an anti-senescence compound that specifically binds to the TAD of the p53 protein or a pharmaceutical composition comprising said anti-senescence compound, preferably a compound or pharmaceutical composition according to the present invention as described herein, b) detecting the amount of phosphorylation of amino acids in the TAD of the p53 protein in a biological sample obtained from said subject and/or detecting the amount of the promyelocytic leukemia protein (PML) bodies in a biological sample obtained from said subject, and c) comparing the amount(s) as detected in step b) with the amount in an earlier sample taken from said subject, and/or a control sample. Preferred is the method wherein an increase of the amount phosphorylation and/or PML bodies is indicative for the success of, progress of and/or sensitivity for the anti-senescence treatment or prophylaxis in the mammalian subject. The method may further comprise adjusting the treatment or prophylaxis of said mammalian patient based on said monitoring. The attending physician will be readily able to make and apply respective treatment decisions, also taking into account additional patient parameters, if required.

Similarly, yet another aspect of the present invention then relates to a method, such as a diagnostic method, for predicting or prognosing the success of, progress of and/or sensitivity for an anti-senescence treatment or prophylaxis in a mammalian subject, comprising performing the method according to the present invention as disclosed herein, wherein an increase of the phosphorylation and/or PML bodies is indicative for the success of, progress of and/or sensitivity for the anti-senescence treatment or prophylaxis in the mammalian subject. As described herein, it was surprisingly found by the inventors that the phosphorylation of the TAD of p53, and/or the amount of PML bodies is/are directly related to the success of, progress of and/or sensitivity for the anti-senescence treatment or prophylaxis.

Preferred is the method according to the present invention, wherein said mammalian subject is a human, mouse, rat, monkey, sheep, goat, hamster, dog, or cat.

Preferred is the method according to the present invention, wherein the anti-senescence treatment or prophylaxis in the mammalian subject comprises administering an effective amount of the compound as identified herein, a pharmaceutical composito as described, a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124 and CL04177, and TAD2 binding derivatives or fragments thereof.

As mentioned herein, the compound is administered to said subject in an effective dosage. This dosage can vary within wide limits and is to be suited to the individual conditions in each individual case. For the above uses, the appropriate dosage will vary depending on the mode of administration, the particular condition to be treated and the effect desired. In general, however, satisfactory results are achieved at dosage rates are as above, e.g., of about 1 to 100 mg/kg animal body weight particularly 1 to 50 mg/kg. Suitable dosage rates for larger mammals, for example humans, are of the order of from about 10 mg to 3 g/day, conveniently administered once or in divided doses, e.g., 2 to 4 times a day, or in sustained release form. In general, a daily dose of approximately 10 mg to 100 mg, particularly 10 to 50 mg, per human individual is appropriate in the case of the oral administration. An effective concentration to be reached at the cellular level can be set at between about 0.05 to 200 $\mu$M, preferably at about 0.01 to 100 $\mu$M, more preferred at btween about 0.05 to 10 $\mu$M. Particularly preferred is topical application, such as to the airways by inhalation. In these cases, the dosage can be conveniently reduced to between 0.1 to 10 mg/dose, preferably 0.2 to 5 mg per dose, which equals about 3 to about 80 $\mu$g per kilogram for a 70 kg subject.

Preferred is the method according to the present invention, wherein the phosphorylation of said TAD2 is positioned at Ser46 and/or Thr55 of human p53, or the analog positions in other mammalian p53 TADs, as also explained further herein. Preferably, the phosphorylation of said TAD2 is positioned at Thr55 of human p53, or the analog positions in other mammalian p53 TADs. Also in this aspect of the invention, further preferred is the method according to the present invention, furthermore comprising detecting the amount of phosphorylation of Ser392 of human p53 or the analog positions in other mammalian p53 proteins, and comparing the amount with the amount in an earlier sample taken from said subject, and/or a control sample, wherein an increase of the phosphorylation is further indicative for the success and/or progress of the anti-senescence treatment or prophylaxis in the mammalian subject.

Peferred is the method according to the present invention, wherein the biological sample obtained from said subject comprises senescent cells, tumor cells, metastatic cells, cells of micro-metastases, and/or fibrotic cells, in particular breast cancer, lung cancer, and/or liver cancer cells or metastatic cells thereof.

Further preferred is the method according to the present invention, wherein the subject further receives an anti-senescent cell chemotherapy, such as an anti-cancer chemotherapy.

Yet another aspect of the present invention then relates to an improved anti-senescence compound that specifically binds to the TAD, preferably the TAD2, of a mammalian p53 protein or a pharmaceutical composition comprising said anti-senescence compound, preferably a compound or pharmaceutical composition according to the present invention for use in the prophylaxis or treatment of senescent cells, wherein the senescent cells exhibit an increase of the amount of phosphorylation of Ser46 and/or Thr55 of human p53, or the analog positions in other mammalian p53 TADs, and/or an increase of the amount of PML protein, when compared to a control cell. Preferably, the phosphorylation of said TAD2 is positioned at Thr55 of human p53, or the analog positions in other mammalian p53 TADs.

Also in this aspect of the invention, further preferred is the improved anti-senescence compound or a pharmaceutical composition for use according to the present invention, wherein the senescent cells further exhibit an increase of the amount of phosphorylation of Ser392 of human p53 or the analog positions in other mammalian p53 proteins, when compared to a control cell.

Peferred is the improved anti-senescence compound or a pharmaceutical composition for use according to the present invention, wherein the biological sample obtained from said subject comprises senescent cells, tumor cells, metastatic cells, cells of micro-metastases, and/or fibrotic cells, in particular breast cancer, lung cancer, and/or liver cancer cells or metastatic cells thereof. Consequently, preferred is the improved anti-senescence compound or a pharmaceutical composition for use according to the present invention, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof.

Further preferred is the improved anti-senescence compound or a pharmaceutical composition for use according to the present invention, wherein the subject further receives an anti-senescent cell chemotherapy, such as an anti-cancer chemotherapy.

Yet another aspect of the present invention then relates to a method for preventing or treating senescent cells in a subject, wherein the senescent cells exhibit an increase of the amount of phosphorylation of Ser46 and/or Thr55 of human p53, or the analog positions in other mammalian p53 TADs, and/or an increase of the amount of PML protein, when compared to a control cell, comprising administering to said subject an effective amount of an anti-senescence compound that specifically binds to the TAD of the p53 protein or a pharmaceutical composition comprising said anti-senescence compound, preferably a compound or pharmaceutical composition according to the present invention. Preferably, the phosphorylation of said TAD2 is positioned at Thr55 of human p53, or the analog positions in other mammalian p53 TADs.

Also in this aspect of the invention, further preferred is the method according to the present invention, wherein the senescent cells further exhibit an increase of the amount of phosphorylation of Ser392 of human p53 or the analog positions in other mammalian p53 proteins, when compared to a control cell.

Peferred is the method according to the present invention, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof. Consequently, preferred is the method according to the present invention, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof. Further preferred is the method according to the present invention, wherein the subject further receives an anti-senescent cell chemotherapy, such as an anti-cancer chemotherapy.

In the medical use aspects of the present invention, the compound (for use) can be provided and/or is administered as a suitable pharmaceutical composition, such as a tablet, capsule, injection, granule, powder, sachet, reconstitutable powder, dry powder inhaler, inhalation, and/or chewable. Such solid formulations may comprise excipients and other ingredients in suitable amounts. Such solid formulations may contain e.g., cellulose, cellulose microcrystalline, polyvidone, in particular FB polyvidone, magnesium stearate and the like. Administration, however, can also be carried out rectally, e.g., in the form of suppositories, or parenterally, e.g., in the form of injections or infusions, or percutaneously, e.g., in the form of ointments, creams or tinctures. Preferred is administration using a dry powder inhaler or other form of inhalation.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e., in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e., ,directly and/or indirectly, preferably synergistically) with the present compound for use.

Thus, the compounds of the invention can be used alone or in combination with other active compounds - for example with medicaments already known for the treatment of the aforementioned diseases, whereby in the latter case a favorable additive, amplifying or preferably synergistically effect is noticed. Suitable amounts to be administered to humans range from 1 to 500 mg, in particular 5 mg to 100 mg, such as between 1 and 10 mg/kg/day oral dose. An effective concentration to be reached at the cellular level can be set at between 0.005 to 200 $\mu$M, as indicated above, preferably at about 0.5 to 10 $\mu$M. Particularly preferred is topical application, such as to the airways by inhalation. In these cases, the dosage can be conveniently reduced to between 0.1 to 10 mg/dose, preferably 0.2 to 5 mg per dose, which equals about 3 to about 80 $\mu$g per kilogram for a 70 kg subject.

Yet another aspect of the present invention then relates to a diagnostic kit comprising materials for performing a method according to the present invention in one or separate containers, optionally together with auxiliary agents and/or instructions for performing said method.

Preferred is a diagnostic kit according to the present invention comprising at least one of an anti-senescence candidate molecule, a recombinantly expressed TAD of a mammalian p53 protein, in particular an N-terminal fragment comprising TAD1 or TAD2, preferably bound or conjugated to a solid carrier.

The kit may further comprise FOXO4 or the TAD binding part thereof, relevant antibodies binding to the coponents of the kit, radiolabeled 32 P-orthophosphate, phospho-specific antibodies, dyes and other labels, as well buffers and matrices for performing the methods as above.

The kit may be used in the methods of the invention, i.e., for identifying an anti-senescence compound, for monitoring an anti-senescence treatment or prophylaxis in a mammalian subject in need thereof, and/or for predicting or prognosing the success of, progress of and/or sensitivity for an anti-senescence treatment or prophylaxis in a mammalian subject.

The present invention relates to the following items.

Item 1. A method for identifying an improved anti-senescence compound, comprising the steps of: a) contacting at least one anti-senescence candidate molecule with a trans activation domain (TAD) of a mammalian p53 protein, b) detecting a specific binding of the candidate molecule to the TAD in the presence of at least one phosphorylated amino acid in the TAD, and c) comparing said specific binding to the binding in the absence of the at least one phosphorylated amino acid in the TAD, wherein an increase of the binding in the presence of the at least one phosphorylated amino acid in the TAD identifies an improved anti-senescence compound.,

Item 2. The method according to Item 1, wherein said contacting is in vivo or in vitro, in solution or comprises the TAD of a mammalian p53 protein or the anti-senescence candidate molecule bound or conjugated to a solid carrier.

Item 3. The method according to Item 1 or 2, wherein said anti-senescence candidate molecule is selected from is a chemical molecule, a molecule selected from a library of small organic molecules, a molecule selected from a combinatory library, a cell extract, in particular a plant cell extract, a small molecular drug, a protein, a protein fragment, a molecule selected from a peptide library, an antibody or fragment thereof, in particular a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124 and CL04177.

Item 4. The method according to any one of Items 1 to 3, wherein detecting said binding comprises detecting phosphorylation comprising radiolabeled 32 P-orthophosphate, phospho-specific antibodies, and/or mass spectrometry.

Item 5. The method according to any one of Items 1 to 4, wherein the TAD of the mammalian p53 protein is selected from TAD1 and/or TAD2 of human, mouse, rat, monkey, sheep, goat, hamster, dog, and cat p53 protein, optionally as part of the full-length p53 protein, in particular a recombinant p53 protein, a full-length p53 protein comprising phosphorylation at Ser392 of human p53 or the analog positions in other mammalian p53 proteins, a recombinant fusion protein comprising said TAD1 and/or TAD2, or a phosphorylated fragment of the TAD1 or TAD2, and mutated variants thereof.

Item 6. The method according to any one of Items 1 to 5, wherein the phosphorylation of said TAD1 domain is positioned at Ser15 and/or Ser20 of human p53, and/or the phosphorylation of said TAD2 is positioned at Ser46 and/or Thr55 of human p53, or the analog positions in other mammalian p53 TADs, preferably, the phosphorylation of said TAD2 is positioned at Thr55 of human p53, or the analog positions in other mammalian p53 TADs.

Item 7. The method according to any one of Items 1 to 6, wherein detecting the binding comprises a competitive binding with the protein FOXO4 or a TAD binding fragment thereof.

Item 8. The method according to any one of Items 1 to 7, wherein detecting the binding furthermore comprises, when possible, detecting phosphorylation of Ser392 of human p53 or the analog positions in other mammalian p53 proteins, and comparing said specific binding to the absence of said phosphorylated amino acid.

Item 9. An anti-senescence compound as identified according to a method according to any one of Items 1 to 8, or a pharmaceutical composition comprising said anti-senescence compound, together with a pharmaceutically acceptable carrier.

Item 10. A method for monitoring an anti-senescence treatment or prophylaxis in a mammalian subject in need thereof, comprising a) providing an anti-senescence treatment or prophylaxis to said subject, comprising administering to said subject an anti-senescence compound that specifically binds to the TAD of the p53 protein or a pharmaceutical composition comprising said anti-senescence compound, preferably a compound or pharmaceutical composition according to Item 9, b) detecting the amount of phosphorylation of amino acids in the TAD of the p53 protein in a biological sample obtained from said subject and/or detecting the amount of the promyelocytic leukemia protein (PML) bodies in a biological sample obtained from said subject, and c) comparing the amount(s) as detected in step b) with the amount in an earlier sample taken from said subject, and/or a control sample.

Item 11. A method for predicting or prognosing the success of, progress of and/or sensitivity for an anti-senescence treatment or prophylaxis in a mammalian subject, comprising performing the method according to claim 10, wherein an increase of the amount phosphorylation and/or PML protein bodies is indicative for the success of, progress of and/or sensitivity for the anti-senescence treatment or prophylaxis in the mammalian subject.

Item 12. The method according to Item 10 or 11, wherein said mammalian subject is a human, mouse, rat, monkey, sheep, goat, hamster, dog, or cat.

Item 13. The method according to any one of Items 10 to 12, wherein the anti-senescence treatment or prophylaxis in the mammalian subject comprises administering an effective amount of a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124 and CL04177, and TAD2 binding derivatives or fragments thereof.

Item 14. The method according to any one of Items 10 to 13, wherein the phosphorylation of said TAD2 is positioned at Ser46 and/or Thr55 of human p53, or the analog positions in other mammalian p53 TADs, preferably, the phosphorylation of said TAD2 is positioned at Thr55 of human p53, or the analog positions in other mammalian p53 TADs.

Item 15. The method according to any one of Items 10 to 14, furthermore comprising detecting the amount of phosphorylation of Ser392 of human p53 or the analog positions in other mammalian p53 proteins, and comparing the amount with the amount in an earlier sample taken from said subject, and/or a control sample, wherein an increase of the phosphorylation is further indicative for the success and/or progress of the anti-senescence treatment or prophylaxis in the mammalian subject.

Item 16. The method according to any one of Items 10 to 15, wherein the biological sample obtained from said subject comprises senescent cells, tumor cells, metastatic cells, cells of micro-metastases, and/or fibrotic cells, in particular breast cancer, lung cancer, and/or liver cancer cells or metastatic cells thereof.

Item 17. The method according to any one of Items 10 to 16, wherein the subject further receives an anti-senescent cell chemotherapy, such as an anti-cancer chemotherapy.

Item 18. An anti-senescence compound that specifically binds to the TAD of the p53 protein or a pharmaceutical composition comprising said anti-senescence compound, preferably a compound or pharmaceutical composition according to claim 9 for use in the prophylaxis or treatment of senescent cells, wherein the senescent cells exhibit an increase of the amount of phosphorylation of Ser46 and/or Thr55 of human p53, or the analog positions in other mammalian p53 TADs, and/or an increase of the amount of PML protein bodies, when compared to a control cell.

Item 19. The anti-senescence compound or a pharmaceutical composition for use according to Item 18, wherein the senescent cells further exhibit an increase of the amount of phosphorylation of Ser392 of human p53 or the analog positions in other mammalian p53 proteins, when compared to a control cell.

Item 20. The anti-senescence compound or a pharmaceutical composition for use according to Item 18 or 19, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof.

Item 21. A method for preventing or treating senescent cells in a subject, wherein the senescent cells exhibit an increase of the amount of phosphorylation of Ser46 and/or Thr55 of human p53, or the analog positions in other mammalian p53 TADs, and/or an increase of the amount of PML protein, when compared to a control cell, comprising administering to said subject an effective amount of an anti-senescence compound that specifically binds to the TAD of the p53 protein or a pharmaceutical composition comprising said anti-senescence compound, preferably a compound or pharmaceutical composition according to Item 9.

Item 22. The method according to Item 21, wherein the senescent cells further exhibit an increase of the amount of phosphorylation of Ser392 of human p53 or the analog positions in other mammalian p53 proteins, when compared to a control cell.

Item 23. The method according to Item 21 or 22, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof.

Item 24. A diagnostic kit comprising materials for performing a method according to any one of Items 1 to 8, and 10 to 17 in one or separate containers, optionally together with auxiliary agents and/or instructions for performing said method.

Item 25. Use of the kit according to Item 24 in a method according to any one of Items 1 to 8, and 10 to 17 for identifying an anti-senescence compound, for monitoring an anti-senescence treatment or prophylaxis in a mammalian subject in need thereof, and/or for predicting or prognosing the success of, progress of and/or sensitivity for an anti-senescence treatment or prophylaxis in a mammalian subject.

The invention will now be further described in the following examples and with reference to the accompanying figures and the sequence listing, without being limited thereto. For the purposes of the present invention, all references as cited herein are incorporated by reference in their entireties.

Figure 1 shows a schematic overview of the domain organization of human p53 protein, with the N-terminally located TADs containing amino acids that may be phosphorylated (S15, S20, S46, and T55), and the C-terminal regulatory domain comprising amino acid S392, which may be phosphorylated as well.

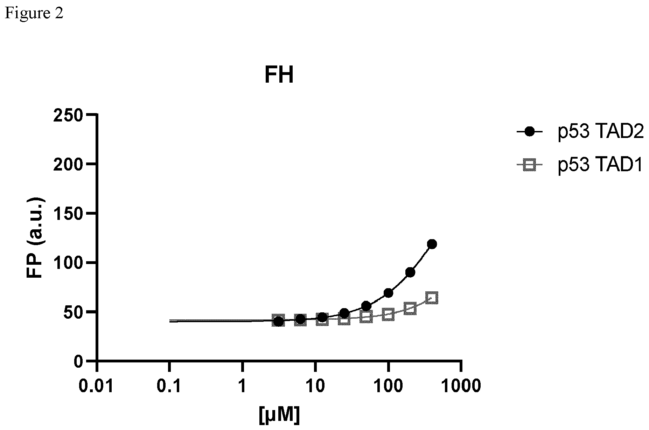

Figure 2 shows the results of a fluorescence polarization binding assay showing binding of the recombinant FOXO4 forkhead domain to FITC-labeled peptides of p53 harboring either the transcription activation domain 1 (p53TAD1) or transcription activation domain 2 (p53$^{TAD2}$).

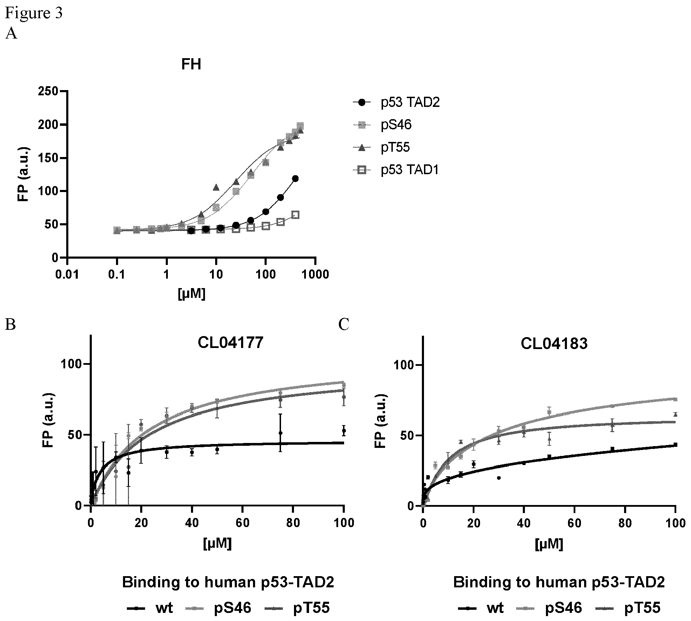

Figure 3 shows the results of a binding assay showing binding of FOXO4 (A) and FOXO4-derived (e.g., mimetic) peptides (B) and (C) with substantially higher affinity to p53-TAD2 when phosphorylated at T55 or S46 (squares and triangles in A, top two graphs in B and C, respectively).

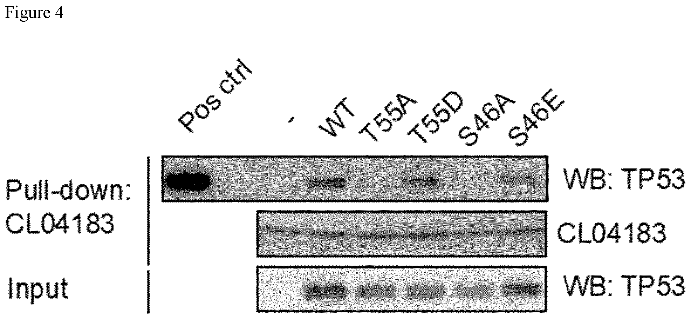

Figure 4 shows that FOXO4-derived peptide CL04183 can bind full-length p53 from cells when it is in the wildtype form or phospho-mimicking on T55 or S46, but not when these sites can not be phosphorylated.

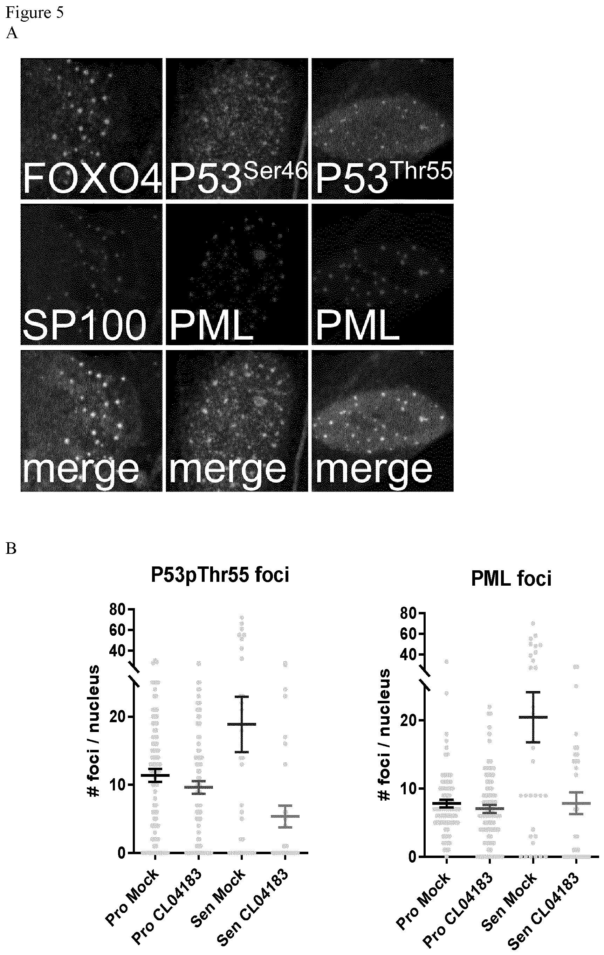

Figure 5 shows immunocytochemistry images of individual nuclei (DNA stained with Hoechst 33258 in blue) for PML bodies (marked by PML and SP100), FOXO4 and phosphorylated p53. p53 phosphorylated on serine 46 or threonin 55 co-localizes with FOXO4 within/directly adjacent to PML bodies. Therefore, PMLs bodies are a proxy for the phosphorylated pool of p53 and FOXO4, and thus helpful as phospho-TP53 stainings on tissues are technically laborious to detect.

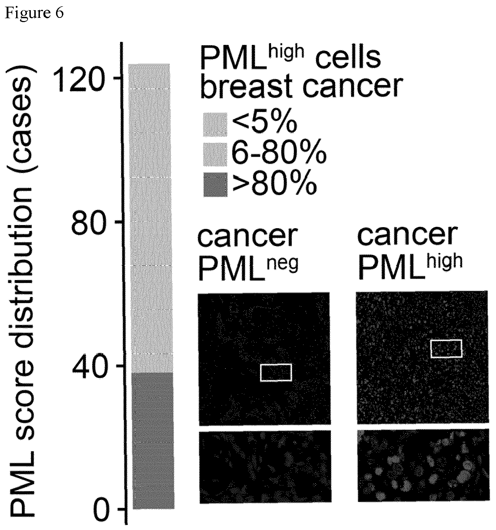

Figure 6 shows immunohistochemistry images for biopsies from human breast cancers with nuclei (DNA stained with Hoechst 33258) in dark gray and PML in light gray, indicating high levels of nuclear PML bodies, both in the cancer cells themselves (about a third of patient biopsies in this cohort).

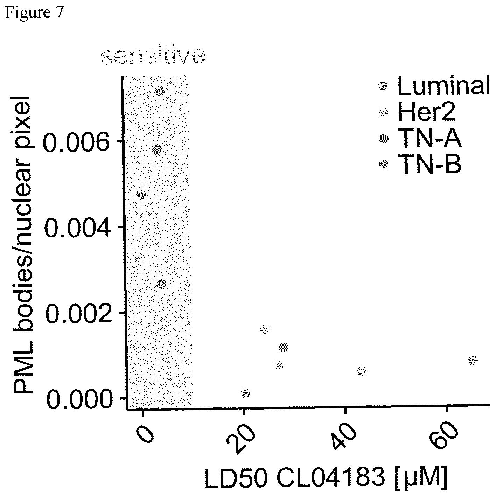

Figure 7 shows the number of PML bodies plotted against the sensivity to FOXO4-derived peptide CL04177 in human breast cancer cell lines grouped by their molecular subtype. Out of the tested cell lines, all with high levels of PML bodies fall under the triple-negative subtype (showing low levels of progesterone receptor, estrogen receptor and human epidermal growth factor receptor 2) and are highly sensitive to compound CL04177, whereas breast cancer lines with few PML bodies are less sensitive.

Figure 8 shows that CL04183 and CL04177 specifically counteract liver metastases. CL04183 primarily eliminates micro-metastases.

Figure 9 shows that CL04183 and 5-FU and combinations thereof specifically counteract liver metastases.

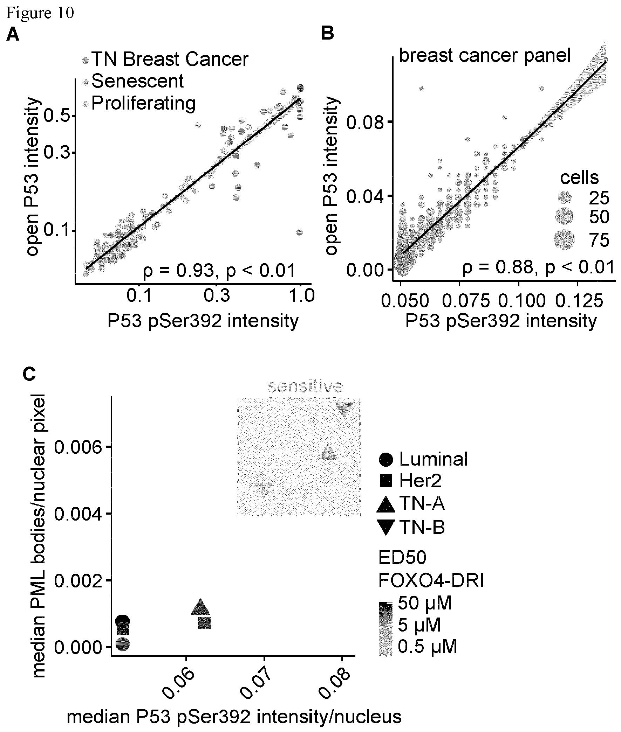

Figure 10 shows A) Correlation of open P53 (Pab240 clone) and P53 pSer392 (EP155Y clone) staining in proliferating and senescent hTERT immortalized retinal pigment epithelium cell line RPE1 as well as human triple-negative breast cancer line MDA-MB-231. Spearman's rank correlation coefficient (0.93) given; B) Correlation of open P53 (Pab240 clone) and P53 pSer392 (EP155Y clone) staining in a panel of eight human breast cancer lines including luminal, Her2 positive and triple-negative. Spearman's rank correlation coefficient (0.88) given; and C) Association between PML body count/nuclear pixel (to correct for variation in nucleus size between lines) and nuclear P53 pSer392 staining intensity in eight human breast cancer lines plotted against the effective dose inducing cell death in 50% of the population (ED50, as measured by MTS assay) after treatment with CL04177 for these cell lines. Sensitive cell lines are highlighted by the box.

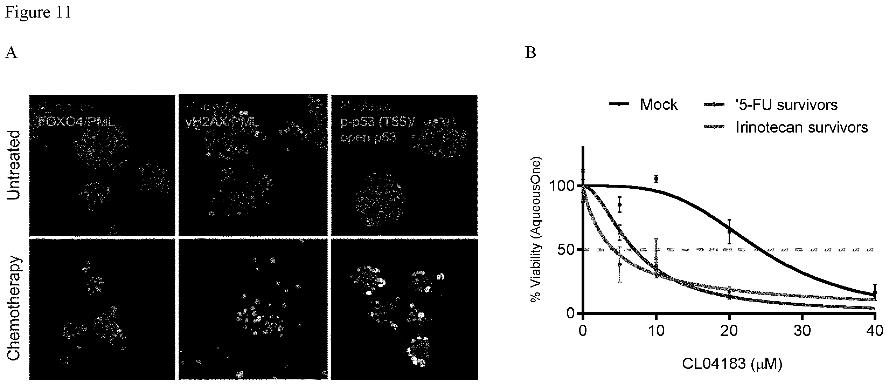

Figure 11 shows that cancer cells that survive chemotherapy show increased PML bodies and phosphorylated p53. These cells are more sensitive to CL04183 treatment. A) The patient-derived colorectal cancer organoid line CRC29 was treated with '5-FluoroUracil (5-FU) for 1 week. Subsequently, an immunofluorescence staining was performed for the indicated proteins. B) CRC29 organoids were treated with '5-FU or Irinotecan in a 96-well plate. Compound CL04183 was added in increasing concentrations to the cells 3 days later and cell viability was measured on day 8.

## EXAMPLES

### Assay and curve-fitting to generate LD50

To study the selectivity of different therapies for cancer cells, colorimetric-based cytotoxicity assays were performed two days following treatments. Cells were cultured in 96-wells plates with 4000 cells per well. These were treated with a serial dilution of compounds in triplicates and mock-treatments (FBS, since peptides were dissolved therein). The AQeousOne Solution Cell Proliferation Assay Kit was used to assess cell viability, according to the manufacturer's instructions. In short, the old medium was removed and 100 µl of fresh medium and 10 µl of CellTiter AQueousOne solution were added to each well. The plates were incubated for 1 hour at 37 °C. The resulting colorimetric reactions were read at 490 nm on a Microplate Absorbance Reader. Relative survival was determined by normalization of the results to Mock ( =100% alive). Drug-response curves were generated using the GraphPad 9.2.0 software by performing nonlinear regression (curve fit) with 4parametersr assuming a standard Hill equation (chosen method: inhibitor or agonist respectively vs. Response, constrain top 100). Lethal dose 50 (LD50) was determined as the concentration at which the drugs lower viability in the MTS assay by 50%.

**Immunocytochemistry staining**

Cell lines were grown on 12 mm diameter glass coverslips in a 24-well plate. They were washed gently with phosphate-buffered saline (PBS) and fixed for 30 minutes with 4% paraformaldehyde (PFA) in PBS (v/v) at 4°C. Afterwards, cells were washed twice with pH 7.0 Tris-buffered saline (TBS) and permeabilized for 2 minutes with 0.1% Triton X-100 (v/v) in TBS followed by blocking with a solution of the 2% w/v secondary antibody-appropriate sera (e.g., 2% horse serum and 2% goat serum) and 2% bovine serum albumin in pH 7.0 TBS (TBSB) for 30 minutes at room temperature. Subsequently, coverslips were placed on parafilm over 50 μl droplets of primary antibodies diluted in TBSB and incubated overnight at 4°C. After washing three times with TBSB, the coverslips were incubated with secondary antibodies and 5 μg/ml Hoechst 33258 for 1 hour in the dark at room temperature. Afterward, the coverslips were washed with distilled water and mounted on glass slides.

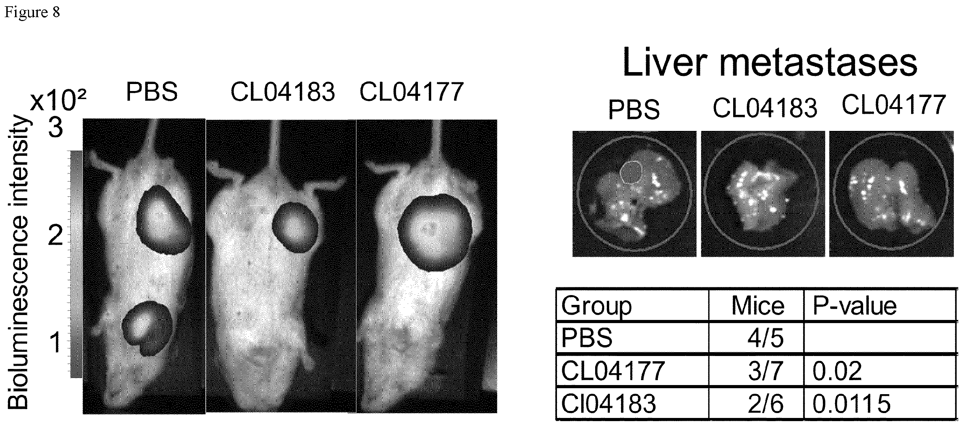

**Anti-metastatic activity of FOXO4-modulating peptides *in vivo* in an othotopic TNBC model (see also Figure 8)**

PBS-treated mice developed metastatic growth visible by bioluminescence. At the same time, treating mice with the CL04183 and CL04177 peptides decreased the incidence of visible metastasis. Treating mice with the two peptides decreased the incidence of liver metastasis as evidenced by the bioluminescence of the tumor cells in the extracted livers.

Approval for this study was obtained by the local University Animal Experimental Committee at the University Medical Center Utrecht (IVD-UMC; WP numbers 9124-1-02 and -05). MDA-MB-231 firefly luciferase-containing cells were transplanted orthotopically into the exposed inguinal mammary fat pad as single cells at a density of 1 millionn cells in 50 ul of a medium into NSG mice (NOD.Cg-Prkdc<scid>IL2rgmWij/Szj) (stock 005557). Carprofen was injected s.c. as analgesia following the surgery. Animals were purchased from Charles River and maintained in a pathogen-free environment.

Tumor growth dynamics were quantified in mice with established tumors (>50mm3). Mice with tumors of 100-200 mm3 in volume were treated daily with peptide (2.5 mg/kg for CL04183 and 10 mg/kg for CL04177) or PBS subcutaneously for one week. After 2 weeks of recovery, mice were sacrificed. For *in vivo* dosing, the peptides were dissolved in PBS. We used 11 mice per group that were randomly assigned to the different treatment groups before the start of the experiment.

To visualize tumor burden in a live mouse, mice were injected with luciferin substrate i.p. at 150 mg/kg, and imaged under isoflurane anesthesia 15 min later using a bioluminescence imager. The M3 Vision software was used to analyze the bioluminescence signal by taking a region of interest (ROI) around the primary tumor to evaluate the BL signal from the tumor or around the upper abdomen and chest of the mouse to evaluate the signal from metastases. The signal was measured in photons/s/cm2.

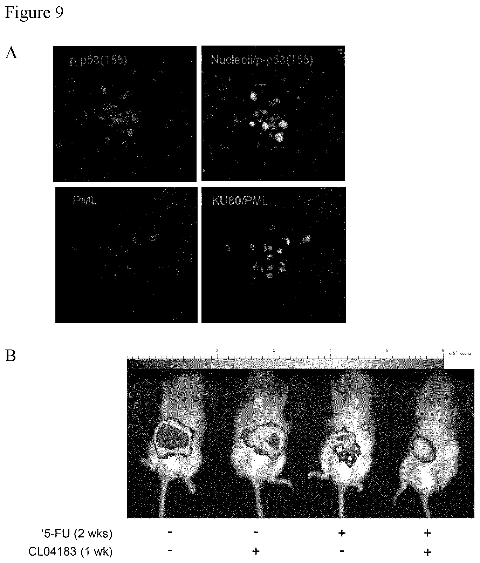

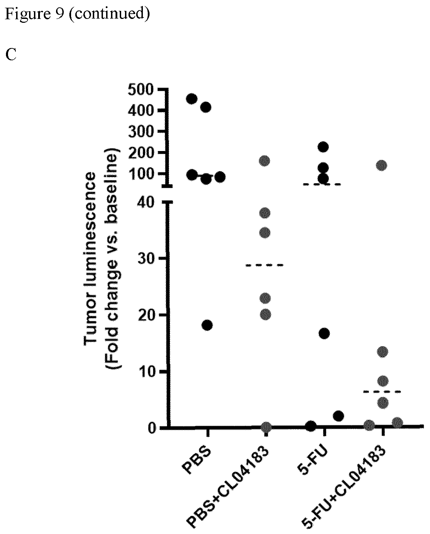

**CL04183 and '5-Fluorouracil reduce colorectal cancer metastasis in liver (see also Figure 9)**

The colorectal cancer organoid line CRC29 was implanted in the caecum of immunodeficient mice. Two weeks after transplantation the animals were treated with either '5-Fluorouracil (5-FU) or PBS. One week after chemotherapy treatment the mice were treated with 3 doses of CL04183 or PBS. A) The livers were stained for KU80 or human nucleoli to detect human cancer cells in the mouse organs, together with p-p53 (T55) or PML. B) The number of liver metastases per stained section was counted by eye.

Mouse experiments were performed after approval from the Dutch animal ethics committee (WP number 9124-1-03). NOD.Cg-Prkdcscid Il2rgtm1Wjl/SzJ/J mice were purchased from Charles River and maintained in a pathogen-free environment. The animals were anaesthetized using 75 mg/kg ketamine and 0.5 mg/kg dexmedetomidine injected i.p., while 5 mg/kg carprofen was injected s.c. as analgesia. Subsequently, CRC29 organoids (250000 cells) containing a firefly luciferase construct were transplanted into the caecum in a 10ul collagen droplet. Two weeks after tumor cell implatation the animals were treated with either 50mg/kg '5-Fluorouracil (5-FU) or PBS. One week after chemotherapy treatment the mice were treated with 3 doses of 2.5mg/kg CL04183 or PBS. Four weeks after transplantation, the mice were sacrificed and tissues were collected for further processing.

**Cell culture**

Breast cancer cell lines (triple-negative: BT549, BT20, Sum149T, MDA-MB-468, MDA-MB-231; Her2+: ZR7530, SKBR3, MDA-MB-361; luminal: MCF7, T47D) and the hTERT-immortalized retinal pigment epithelium cell line RPE1 were cultured in Dulbecco's Modified Eagle's Medium containing 4.5 g/mL glucose, 4 mM glutamine, 10% fetal calf serum, 100 units of potassium penicillin/mL and 100 units of streptomycin sulfate/mL at 37 °C, 5% $CO_2$ and 5% oxygen in a humidified incubator.

**Immunohistochemistry**

Sections of paraffin-embedded, formalin fixated tissues were rehydrated in decreasing concentrations of xyleen and ethanol before being washed in pH 7.0 Tris-buffered saline (TBS) and boiled for 20 minutes in 1 mM EDTA in TBS at pH 9.4 for antigen unmasking. After the slides were left to cool for 30 minutes, the tissue was permeabilized with 0.2% TX-100 in TBS for 5 minutes at room temperature. Subsequently, the sections were washed with TBS and incubated for 1 hour in blocking buffer containing 2% w/v secondary antibody-appropriate sera (e.g., donkey or goat) and 0.1% fish gelatin in 1% BSA. The sections were then encircled with a water-repellent pen and incubated with the primary antibody diluted in TBS/1% BSA overnight at 4°C. The next day, the tissues were washed 3 times with TBS before an hour incubation with fluorescent labelled secondary antibody diluted in blocking buffer (containing nuclear staining with Hoechst 33258). Subsequently, the slides were washed twice in TBS, incubated in Sudan black solution for 20 min to reduce background and washed in demineralized water. The sections were then mounted with Vectashield and imaged using a LSM880 Zeiss confocal microscope.

**Organoid culture**

Organoids were grown at 37 °C and 5% $CO_2$ in a humidified incubator. All organoids were cultured in matrigel (Corning) droplets in advanced DMEM/F12 (Lonza) supplemented with 1% glutamax, 1% Penicillin/Streptomycin, 1% (10 mM) HEPES, 10% Noggin conditioned medium, 2% B-27 (50X; (Thermo/Life Technologies), N-acetylcysteine ((Sigma-Aldrich, 1.25 mM)), A83-01 (Tocris, 500 nM) and SB203580 (Invitrogen / Life Technologies, 3 $\mu$M). To perform viability and apoptosis assays, the organoids were passaged through resuspension in ice-cold medium, followed by centrifugation in 15ml tubes at 4°C. The resulting pellet was trypsinized for 5 minutes at 37 °C to obtain single cells and subsequently washed twice with advanced DMEM/F12 media. These cells were then resuspended in Matrigel and plated in 96-well plates in 5ul droplets. 100ul fresh medium was added to the wells 15 minutes later. Peptide and chemotherapy treatment was added to the organoids 2 days after plating.

**MTS assay**

Six days after treatment, cells were incubated with 10ul CellTiter 96® AQueous One Solution Cell Proliferation Assay (Promega) for 1 hour at 37°C to perform an MTS assay. Subsequently, absorbance was measured at 490nm using a Spectramax M5e.

**Immunofluorescence organoids**

CRC29 organoids were fixed in 4% w/v paraformaldehyde [PFA] for 45 minutes at 4°C, and subsequently washed in 1% w/v BSA in TBS. The organoids were then permeabilized with 0.1% TX-100 in TBS for 5 minutes at room temperature, washed again and and incubated for 1 hour at 4 °C in blocking buffer containing 2% w/v secondary antibody-appropriate sera (e.g., donkey or goat) and 0.1% fish gelatin in 1% BSA. After blocking, the organoids were incubated with the primary antibody diluted in blocking buffer overnight at 4°C. The next day, the organoids were washed twice for an hour at 4°C, before overnight incubation at 4°C with fluorescent-labelled secondary antibodies diluted in blocking buffer. Subsequently, the organoids were washed twice again for an hour at 4°C, with Hoechst 33342 added to the second wash. The stained organoids were then dissolved in glycerol-fructose clearing solution and imaged using a LSM880 Zeiss confocal microscope.

**Protein expression and purification**

Expression constructs for the fragments of human FOXO4 (Uniprot ID P98177) from amino acid 86 to 207 (FOXO4 forkhead) were generated by synthesis of the corresponding optimized FOXO4 cDNA constructs (Genscript) and insertion of these cDNA into a pETM11-His$_6$-protein A-TEV cleavage site vector via NcoI/BamHI restriction digest. For expression of recombinant unlabeled His$_6$-protein-A tagged FOXO4 forkhead protein, the bacterial expression vectors were transformed into Eschericia Coli BL21-DE3 Star strain and 1 L expression cultures were grown for 2 days in minimal medium supplemented with either 6 g of glucose and 3 g of ammonium chloride (Sigma). Cells were diluted to an OD (600nm) of 0.8 and induced with 0.5 mM IPTG followed by protein expression 16 h at 20°C. Cell pellets were harvested and sonicated in nondenaturing lysis buffer (50 mM Tris-HCl pH 7.5, 150 mM NaCl, 20 mM Imidazole, 2 mM tris(2-carboxyethyl)phosphine). Tagged recombinant proteins were then purified using Ni-NTA agarose (QIAGEN) and the His$_6$-protein A tag was cleaved with TEV protease treatment. Untagged proteins were then isolated performing a second affinity purification using Ni-NTA beads. A final size exclusion chromatography purification step was performed in the buffer of interest on a gel filtration column (Superdex 75, GE Healthcare). Protein concentrations were estimated based on their absorbance at 280 nm, assuming that the $\varepsilon$ at 280 nm was equal to the theoretical $\varepsilon$ value.

**Fluorescence anisotropy studies**

Data was acquired on a ClarioStar Plus plate reader. Endpoint measurement with 200 flashes per well were performed. An excitation filter with 482 nm wavelength and emission filter with 530 nm were used and gain adjustment as well as focal height adjustment were performed for each measurement. Fluorescence intensity, parallel fluorescence polarization and perpendicular fluorescence polarization were recorded. Data analysis was performed using MARS Version 3.4 (BMG), Microsoft Excel and GraphPad Prism Version 8.

For measurements, 116 $\mu$l of a solution of the specific Cleara peptides (ranging from 1 to 100 $\mu$M) were prepared. Subsequently 4 $\mu$l of FITC-labelled p53 peptides (stock concentration 15 $\mu$M, resulting in a final concentration of 500 nM) were added. 35 $\mu$l were then transferred in each well of the 384-well plate. Measurements were performed in triplicates. If necessary, this setup was extended with lower concentrations in order to cover strong interactions.

The anisotropy (r) is defined as the ratio of the polarized component to the total intensity ($I_T$):

$$r = \frac{I_z - I_y}{I_x + I_y + I_z}$$

, where the excitation is polarized along the z-axis, emission from the fluorophore is symmetric around the z-axis, so $I_x = I_y$ and $I_y = I_\parallel$, $I_z = I_\perp$, resulting in:

$$r = \frac{I_\parallel - I_\perp}{I_\parallel - 2I_\perp} = \frac{I_\parallel - I_\perp}{I_T}$$

The obtained anisotropy values for each concentration of CL peptide can be used to determine the binding affinity, by applying following fitting function:

$$Y = \frac{R_0 + B_{max} * x}{(K_d + x)}$$

, where Ro is the initial value, dependent on the measurement (to correct for the offset), $B_{max}$ is the maximal anisotropy, $X$ corresponds to the CL peptide concentration and the $K_d$ is fitted at half of the maximal anisotropy.

**Experiments with triple-negative breast cancer cell lines in order to study "open" p53 (see also Figure 10)**

**Cell culture**

Breast cancer cell lines (triple-negative: BT549, BT20, Sum149T, MDA-MB-468, MDA-MB-231; Her2+: ZR7530, SKBR3, MDA-MB-361; luminal: MCF7, T47D), the hTERT-immortalized retinal pigment epithelium cell line RPE1 as well as the IMR90 normal fetal lung fibroblast strain were cultured in Dulbecco's Modified Eagle's Medium containing 4.5 g/mL glucose, 4 mM glutamine, 10% fetal calf serum, 100 units of potassium penicillin/mL and 100 units of streptomycin sulfate/mL at 37 °C, 5% $CO_2$ and 5% oxygen in a humidified incubator.

**MTS assay**

Six days after treatment, cells were incubated with 10ul CellTiter 96® AQueous One Solution Cell Proliferation Assay (Promega) for 1 hour at 37°C to perform an MTS assay. Subsequently, absorbance was measured at 490nm using a Spectramax M5e.

**Immunocytochemistry**

Breast cancer cell lines and the RPE1 cell line were grown on 12 mm diameter 1.5H glass coverslips in a 24-well plate. They were washed gently with phosphate-buffered saline (PBS) at room temperature and fixed for 30 minutes with 4% paraformaldehyde (PFA) in PBS (v/v) at 4°C. Afterwards, cells were washed twice with tris-buffered saline (TBS )and permeabilized for 2 minutes with 0.1% Triton X-100 (v/v) in TBS followed by blocking with a solution of the 2% w/v secondary antibody-appropriate sera (e.g., 2% horse serum and 2% goat serum) and 2% bovine serum albumin in pH 7.0 TBS (TBSB) for 30 minutes at room temperature. Subsequently, coverslips were placed on parafilm over 50 μl droplets of primary antibodies diluted in TBSB and incubated overnight at 4°C. After washing three times with TBSB, the coverslips were incubated with secondary antibodies and 5 μg/ml Hoechst 33258 for 1 hour in the dark at room temperature. Afterward, the coverslips were washed with distilled water and mounted on glass slides.

**Antibodies**

For open conformation P53, cells were stained with 10 μg/mL of the pAb240 clone antibody raised against P53 (mouse monoclonal, supplied by Abcam of Cambridge UK, catalogue number ab26; RRID:AB_303198) and with 260 ng/ml of the clone EP155Y antibody raised against P53 pSer392 (rabbit monoclonal, supplied by Abcam of Cambridge, UK; catalogue number ab33889; RRID:AB_776988).

**Statistics**

Association between P53 pSer392 and open P53 is given as Spearman's rank correlation coefficient, calculated using the R package stats (ver. 4.1.1, (R Core Team (2018) R: A Language and Environment for Statistical Computing. Vienna, Austria: R Foundation for Statistical Computing. Available at: https://www.r-project.org/)) with the p-values (two-sided alternative hypotheses) calculation based on the AS 89 algorithm (Best, D.J. and Roberts, D.E. (1975) 'Algorithm AS 89: The Upper Tail Probabilities of Spearman's Rho', Applied Statistics, 24(3), p. 377. doi:10.2307/2347111).

**Exemplary (CL) peptides as used**

Preferred embodiments of peptides as used or for use according to the present invention are

CL04124: RKKASSKIEAAILDAFSQNWRFFKRPPRRRQRRKKRGAKIEAAILDAF SQNWRKRRRRQRRKKRG (SEQ ID NO: 1);

CL04177: RKKASSKIEAEILDAFSQNWRRKRPPRRRQRRKKRG (SEQ ID NO: 2); and
CL04183: AKIEAAILDAFSQNWRKRRRRQRRKKRG (SEQ ID NO: 3);
and peptides comprising the following amino acid sequences
KIEAEILDAFSQNWRKR (SEQ ID NO: 4) (Core sequence of CL04177 and CL04124); and
KIEAAILDAFSQNWRKR (SEQ ID NO: 5) (Core sequence of CL04183);
wherein the amino acids in said amino acid sequences as herein are preferably D-amino acid residues.

**Claims**

1. A method for identifying an improved anti-senescence compound, comprising the steps of:

    a) contacting at least one anti-senescence candidate molecule with a trans activation domain (TAD) of a mammalian p53 protein,
    b) detecting a specific binding of the candidate molecule to the TAD in the presence of at least one phosphorylated amino acid in the TAD, and
    c) comparing said specific binding to the binding in the absence of the at least one phosphorylated amino acid in the TAD,
    wherein an increase of the binding in the presence of the at least one phosphorylated amino acid in the TAD identifies an improved anti-senescence compound.

2. The method according to claim 1, wherein said contacting is in vivo or in vitro, in solution or comprises the TAD of a mammalian p53 protein or the anti-senescence candidate molecule bound or conjugated to a solid carrier.

3. The method according to claim 1 or 2, wherein said anti-senescence candidate molecule is selected from a chemical molecule, a molecule selected from a library of small organic molecules, a molecule selected from a combinatory library, a cell extract, in particular a plant cell extract, a small molecular drug, a protein, a protein fragment, a molecule selected from a peptide library, an antibody or fragment thereof, in particular a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124 and CL04177 or the core sequences thereof.

4. The method according to any one of claims 1 to 3, wherein detecting said binding comprises detecting phosphorylation comprising radiolabeled 32 P-orthophosphate, phospho-specific antibodies, and/or mass spectrometry.

5. The method according to any one of claims 1 to 4, wherein the TAD of the mammalian p53 protein is selected from TAD1 and/or TAD2 of human, mouse, rat, monkey, sheep, goat, hamster, dog, and cat p53 protein, optionally as part of the full-length p53 protein, in particular a recombinant p53 protein, a full-length p53 protein comprising phosphorylation at Ser392 of human p53 or the analog positions in other mammalian p53 proteins, a recombinant fusion protein comprising said TAD1 and/or TAD2, or a phosphorylated fragment of the TAD1 or TAD2, and mutated variants thereof.

6. The method according to any one of claims 1 to 5, wherein the phosphorylation of said TAD1 is positioned at Ser15 and/or Ser20 of human p53, and/or the phosphorylation of said TAD2 is positioned at Ser46 and/or Thr55 of human p53, or the analog positions in other mammalian p53 TADs.

7. The method according to any one of claims 1 to 6, wherein detecting the binding furthermore comprises, when possible, detecting phosphorylation of Ser392 of human p53 or the analog positions in other mammalian p53 proteins, and comparing said specific binding to the absence of said phosphorylated amino acid.

8. An anti-senescence compound as identified according to a method according to any one of claims 1 to 7, or a pharmaceutical composition comprising said anti-senescence compound, together with a pharmaceutically acceptable carrier.

9. A method for monitoring an anti-senescence treatment or prophylaxis in a mammalian subject, preferably a human, in need thereof, comprising

a) providing an anti-senescence treatment or prophylaxis to said subject, comprising administering to said subject an anti-senescence compound that specifically binds to the TAD of the p53 protein or a pharmaceutical composition comprising said anti-senescence compound, preferably a compound or pharmaceutical composition according to claim 8,
b) detecting the amount of phosphorylation of amino acids in the TAD of the p53 protein in a biological sample obtained from said subject and/or detecting the amount of the promyelocytic leukemia protein (PML) bodies in a biological sample obtained from said subject, and
c) comparing the amount(s) as detected in step b) with the amount in an earlier sample taken from said subject, and/or a control sample.

10. A method for predicting or prognosing the success of, progress of and/or sensitivity for an anti-senescence treatment or prophylaxis in a mammalian subject, preferably a human, comprising performing the method according to claim 9, wherein an increase of the amount of phosphorylation and/or PML protein bodies is indicative for the success of, progress of and/or sensitivity for the anti-senescence treatment or prophylaxis in the mammalian subject.

11. The method according to claim 9 or 10, wherein the anti-senescence treatment or prophylaxis in the mammalian subject comprises administering an effective amount of a retro-inverso peptide derived from mammalian FOXO4 protein, a fusion peptide comprising a retro-inverso peptide derived from mammalian FOXO4 protein, and derivatives thereof, preferably a peptide selected from CL04183, CL04124, and CL04177, and TAD2 binding derivatives or fragments thereof, such as the core sequences.

12. The method according to any one of claims 9 to 11, wherein the phosphorylation of said TAD2 is positioned at Ser46 and/or Thr55 of human p53, or the analog positions in other mammalian p53 TADs.

13. The method according to any one of claims 9 to 12, furthermore comprising detecting the amount of phosphorylation of Ser392 of human p53 or the analog positions in other mammalian p53 proteins, and comparing the amount with the amount in an earlier sample taken from said subject, and/or a control sample, wherein an increase of the phosphorylation is further indicative for the success and/or progress of the anti-senescence treatment or prophylaxis in the mammalian subject.

14. An anti-senescence compound that specifically binds to the TAD of the p53 protein or a pharmaceutical composition comprising said anti-senescence compound, preferably a compound or pharmaceutical composition according to claim 8 for use in the prophylaxis or treatment of senescent cells, wherein the senescent cells exhibit an increase of the amount of phosphorylation of Ser46 and/or Thr55 of human p53, or the analog positions in other mammalian p53 TADs, and/or an increase of the amount of PML protein bodies, when compared to a control cell, wherein preferably the senescent cells further exhibit an increase of the amount of phosphorylation of Ser392 of human p53 or the analog positions in other mammalian p53 proteins, when compared to a control cell.

15. The anti-senescence compound or a pharmaceutical composition for use according to claim 14, wherein said senescent cells are selected from, tumor cells, metastatic tumor cells, cells of tumor micro-metastases, fibrotic cells, breast cancer cells, such as triple-negative breast cancer cells, lung cancer, and/or liver cancer cells or metastatic cells thereof.

Figure 1

Figure 2

Figure 3

A

B                 C

Figure 4

Figure 5

A

B

Figure 6

Figure 7

Figure 8

## Liver metastases

| Group | Mice | P-value |
|-------|------|---------|
| PBS | 4/5 | |
| CL04177 | 3/7 | 0.02 |
| Cl04183 | 2/6 | 0.0115 |

Figure 9

Figure 9 (continued)

C

Figure 10

Figure 11

A

B

EP 4 249 912 A1

Europäisches
Patentamt

European
Patent Office

Office européen
des brevets

# EUROPEAN SEARCH REPORT

Application Number

EP 22 16 4038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/118014 A2 (UNIV ERASMUS MEDICAL CT [NL]) 28 July 2016 (2016-07-28) | 8,14,15 | INV. G01N33/50 G01N33/68 |
| Y | * page 12, line 28 - page 19, line 29; claims 1-4; figures 2,3 * | 1-15 | |
| X | MARJOLEIN P. BAAR ET AL: "Targeted Apoptosis of Senescent Cells Restores Tissue Homeostasis in Response to Chemotoxicity and Aging", CELL, vol. 169, no. 1, 1 March 2017 (2017-03-01) , pages 132-147.e16, XP055606061, Amsterdam NL ISSN: 0092-8674, DOI: 10.1016/j.cell.2017.02.031 | 8,14,15 | |
| Y | * abstract; figures 1-7 * * page 3 - page 10 * | 1-15 | |
| X | WO 2019/069070 A1 (ETERNANS LTD [GB]) 11 April 2019 (2019-04-11) | 8,14,15 | |
| Y | * paragraph [0100] - paragraph [0104]; claims 1-34 * | 1-15 | TECHNICAL FIELDS SEARCHED (IPC) G01N |
| Y | PUTAVET DIANA ET AL: "Abstract P1-19-02: Repurposing the FOXO4 senolytic against triple-negative breast cancer", CANCER RESEARCH, vol. 82, no. 4_Supplement, 15 February 2022 (2022-02-15), pages P1-19-02-P1-19-02, XP055958750, US ISSN: 0008-5472, DOI: 10.1158/1538-7445.SABCS21-P1-19-02 Retrieved from the Internet: URL:https://aacrjournals.org/cancerres/art icle/82/4_Supplement/P1-19-02/680494/Abstr act-P1-19-02-Repurposing-the-FOXO4-senolyt ic> * the whole document * | 1-15 | |

The present search report has been drawn up for all claims

| Place of search | Date of completion of the search | Examiner |
|---|---|---|
| Munich | 8 September 2022 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 16 4038**

This annex lists the patent family members relating to the patent documents cited in the above-mentioned European search report.
The members are as contained in the European Patent Office EDP file on
The European Patent Office is in no way liable for these particulars which are merely given for the purpose of information.

**08-09-2022**

| Patent document cited in search report | | Publication date | Patent family member(s) | | Publication date |
|---|---|---|---|---|---|
| WO 2016118014 | A2 | 28-07-2016 | AU | 2016209737 A1 | 17-08-2017 |
| | | | BR | 112017015833 A2 | 27-03-2018 |
| | | | CA | 2974623 A1 | 28-07-2016 |
| | | | CL | 2017001883 A1 | 02-02-2018 |
| | | | CN | 107531768 A | 02-01-2018 |
| | | | EP | 3247375 A2 | 29-11-2017 |
| | | | EP | 3936141 A2 | 12-01-2022 |
| | | | JP | 6707549 B2 | 10-06-2020 |
| | | | JP | 2018508196 A | 29-03-2018 |
| | | | PE | 20180128 A1 | 18-01-2018 |
| | | | RU | 2017128116 A | 26-02-2019 |
| | | | SG | 11201706029V A | 30-08-2017 |
| | | | US | 2018015137 A1 | 18-01-2018 |
| | | | WO | 2016118014 A2 | 28-07-2016 |
| WO 2019069070 | A1 | 11-04-2019 | CN | 111417647 A | 14-07-2020 |
| | | | US | 2020255489 A1 | 13-08-2020 |
| | | | WO | 2019069070 A1 | 11-04-2019 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016118014 A
- WO 2013152038 A
- WO 2013152041 A
- WO 2018129007 A
- WO 2021165538 A

### Non-patent literature cited in the description

- **BAKER et al.** *Nature,* 2011, vol. 479 (7372), 232-6
- **BOURGEOIS ; MADL.** Regulation of cellular senescence via the FOXO4-p53 axis. *FEBS Lett.,* June 2018, vol. 592 (12), 2083-2097
- **MILLER JENKINS et al.** p53 N-terminal phosphorylation: a defining layer of complex regulation. *Carcinogenesis,* August 2012, vol. 33 (8), 1441-1449, https://doi.org/10.1093/carcin/bgs145
- **FENG et al.** Ser46 phosphorylation regulates p53-dependent apoptosis and replicative senescence. *Cell Cycle.,* 01 December 2006, vol. 5 (23), 2812-9
- Repurposing the FOXO4 senolytic against triple-negative breast cancer [abstract. **DIANA PUTAVET et al.** Proceedings of the 2021 San Antonio Breast Cancer Symposium. AACR, 07 December 2021
- *Cancer Res,* 2022, vol. 82 (4), 1-19, 02
- **RAJ ; ATTARDI.** The Transactivation Domains of the p53 Protein. *Cold Spring Harb Perspect Med.,* 03 January 2017, vol. 7 (1), a026047
- **CHRISTOPHER D. SPICER ; BENJAMIN G. DAVIS.** *Selective chemical protein modification Nature Communications,* 2014, vol. 5 (4740
- **SAKAMOTO S ; HAMACHI I.** Recent Progress in Chemical Modification of Proteins. *Anal Sci.,* 10 January 2019, vol. 35 (1), 5-27
- Remington's Pharmaceutical Sciences. Mack Publishing Co, 1991
- **BAUER et al.** Pharmazeutische Technologic. Govi-Verlag, 1997
- **R CORE TEAM.** R: A Language and Environment for Statistical Computing. R Foundation for Statistical Computing, 2018
- **BEST, D.J. ; ROBERTS, D.E.** Algorithm AS 89: The Upper Tail Probabilities of Spearman's Rho. *Applied Statistics,* 1975, vol. 24 (3), 377